# EUROPEAN PATENT SPECIFICATION

(11) **EP 2 619 602 B1**
(45) Date of publication and mention of the grant of the patent: **31.10.2018**
(21) Application number: 11764602.6
(22) Date of filing: 21.09.2011
(51) Int. Cl.: G01R 33/28, A61B 10/02, A61B 6/00, A61B 6/03, A61B 6/12

(54) **MR- OR CT-GUIDED MEDICAL INSTRUMENTS**
MR- ODER CT-GEFÜHRTE MEDIZINISCHE INSTRUMENTE
INSTRUMENTS MÉDICAUX GUIDÉS PAR TOMODENSITOMÉTRIE OU PAR RÉSONANCE MAGNÉTIQUE

(30) Priority: 30.09.2010 GB 201016461; 22.09.2010 GB 201015897
(43) Date of publication of application: 31.07.2013
(73) Proprietor: Cook Medical Technologies LLC, Bloomington, IN 47404 (US)
(72) Inventor: COCKBURN, John, Norwich NR4 7UY (GB)
(74) Representative: Garratt, Peter Douglas
(86) International application number: PCT/GB2011/051785
(87) International publication number: WO 2012/038748

(56) References cited:
- WO-A1-2006/082413
- WO-A1-2010/125377
- MÜLLER-BIERL BERND ET AL: "Numerical modeling of needle tip artifacts in MR gradient echo imaging", MEDICAL PHYSICS, AIP, MELVILLE, NY, US, vol. 31, no. 3, 1 March 2004 (2004-03-01), pages 579-587, XP012074808, ISSN: 0094-2405, DOI: 10.1118/1.1640971
- WEERAKKODY R A ET AL: "The air bubble technique for confirming the location of an image-guided biopsy - a technical note.", BRITISH JOURNAL OF NEUROSURGERY JUN 2009 LNKD- PUBMED:19533471, vol. 23, no. 3, June 2009 (2009-06), pages 329-331, XP009155350, ISSN: 1360-046X

## Description

The present invention relates to medical instruments, and in particular to medical instruments, which can be guided into place using magnetic resonance imaging (MRI) or computed tomography (CT). The invention especially relates to penetrating instruments inserted percutaneously (e.g. needles), for use in a wide range of medical applications using MRI or CT, such as tumour localisation (e.g. in the breast), biopsy, drainage, tissue ablation, cannula, catheter or central line insertion, and in cosmetics.

Various imaging modalities, such as ultrasound, computed tomography and magnetic resonance imaging, are used to show body areas which are being targeted for medical treatment. A chosen diagnostic imaging modality would ideally be able to demonstrate the target tissue as well as the medical instrument which is delivering the treatment procedure. Such procedures include, but are not limited to, the deposition of markers into breast tumours which allow a surgeon to excise the correct tissue during a subsequent operation; biopsy of tissues requiring histological characterization, e.g. liver, kidney, lung; ablation of tissues which need to be destroyed, e.g. tumours; and the insertion of drainage tubes for a variety of reasons which include mechanical blockage of organs such as kidney and liver, and the decompression of abscess cavities.

All such procedures require the body tissue to be represented in a way which allows the target site to be easily seen, while also allowing the medical instrument to be seen in contrast. In this way, precise steering towards a target can be achieved quickly, with the minimum number of attempts, and with an accuracy which avoids inadvertent damage to intervening or adjacent non-target structures such as blood vessels, nerves and bowel. The inventor of the present invention has previously demonstrated, in PCT /GB2010/050682, that, using ultrasound, conductive materials, such as metal, can be rendered more visible to the insonant sound waves by applying an electrolytic direct current charge to the material as it is inserted into the body. The result of this is the creation of an 'aura' or cloud of tiny gas bubbles at the surface of the conducting material. These gas bubbles are intensely hyper-reflective ('bright') on ultrasound and they render the material highly visible.

In addition, WO 2006/082413, which concerns a needle assembly for RF ablation of target tissue in the liver of a patient, notes that the electrodes of the needle assembly may, as part of necrotizing the target tissue, induce electrolysis in the target tissue, which may in turn generate bubbles in the region of the electrode, with these bubbles enabling the position of the electrode to be determined using ultrasound imaging.

The present invention relates to the use of electrolysis for rendering materials more visible under other imaging modalities, for example magnetic resonance imaging and computed tomography. Computed Tomography (CT) is a powerful non-destructive evaluation (NDE) technique for producing 2D and 3D cross-sectional images of an object from flat X-ray images. Characteristics of the internal structure of an object, such as dimensions, shape, internal defects, and density are readily available from CT images. The test object, such as a patient's body, is placed on a table that is between a radiation source and an imaging system. The table and the imaging system are connected to a computer so that x-ray images collected can be correlated to the position of the test object. The imaging system produces a 2D image of the specimen. Specialized computer software makes it possible to produce cross-sectional images of the test object as if it was being sliced.

Magnetic resonance imaging (MRI) is primarily a non-invasive medical imaging technique used in radiology to visualize detailed internal structure and limited function of the body. MRI provides much greater contrast between the different soft tissues of the body than CT does, making it especially useful in neurological, musculoskeletal, cardiovascular, and oncological imaging. Unlike CT, MRI uses no ionizing radiation. Instead, it uses a powerful magnetic field to align the nuclear magnetization of hydrogen atoms (i.e. protons) in water in the body. Radio frequency (RF) fields are used to systematically alter the alignment of this magnetization. This causes the hydrogen nuclei to produce a magnetic field detectable by the scanner. This signal can be manipulated by additional magnetic fields to build up enough information to construct an image of the body.

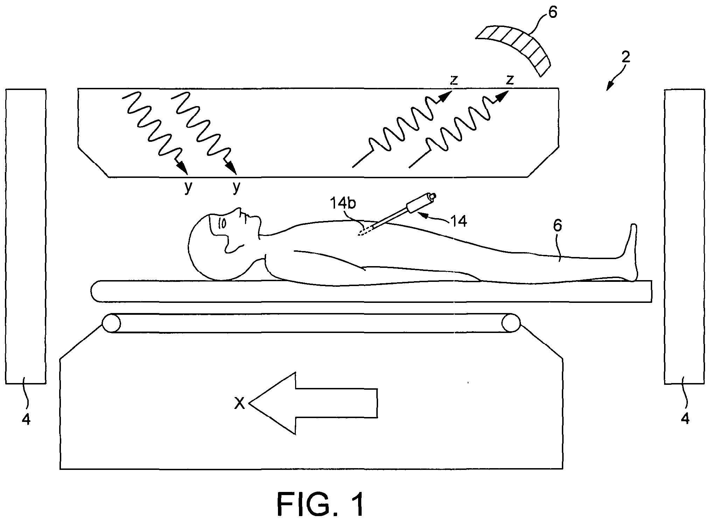

An example of the use of MRI guidance is in the field of breast localization, using an apparatus as shown in Figure 1. During this procedure, the patient lies in an MRI scanner and images are taken which show a breast tumour. A special mesh guide is placed next to the skin which allows accurate depiction of the best route to take in order to insert a localizing wire into the tumour. The physician directs a needle into the breast tumour by inserting it through the skin while the patient is still in the scanner, and, by taking regular images while advancing the needle a short distance at a time, the tip of the needle can be positioned exactly at the desired position. Subsequently, a special hooked wire is advanced through the needle into the tumour. The hook engages the tumour tissue preventing the wire from slipping out. Following this procedure, the patient, with the wire still in place, undergoes surgery. The surgeon uses the localizing wire to find the tumour at operation.

Unfortunately, many breast tumours can be very difficult to see during an operation, and the wire is an essential part of a successful procedure. The problem with this arrangement is that metallic materials create an immense amount of artifact on MRI, which consists of a large, irregular 'black hole' from which no useful MRI signal can be derived. Certain MRI settings can reduce this artefact. However, it is currently extremely difficult to take an MRI image of a metal needle while displaying its dimensions accurately. A metal needle always looks much 'fatter' on MRI than it actually is, and adjacent tissue (e.g. the tumour) is rendered poorly visible or even invisible owing to the artifactual 'black hole' surrounding the needle. Attempts to use non-metallic needles and localizing wires suffer from the opposite problem. They are very poorly visible on MRI because plastics, ceramics and non-watery materials contain no mobile protons, which are required for substance/tissue visibility on MRI.

The existence of MRI artefacts from needles has been discussed in the prior art. For example, Müeller-Bierl et al., in their paper "Numerical modeling of needle tip artifacts in MR gradient echo imaging" (Medical Physics, vol. 31, no. 3, 1 March 2004) note that, for interventional procedures under control of magnetic resonance imaging (MRI), exact needle tip navigation is complicated by the paramagnetism of microsurgical instruments. Specifically, they note that local magnetic field inhomogeneities are induced resulting in position encoding artifacts and in signal voids in the surrounding of instruments and especially near their tips.

The authors carried out systematic numerical studies of the field distribution for variable types of concentric and asymmetric tip shapes, for different ratios between tip length and needle diameter, and for different orientations of the needle axis in the external static magnetic field. Based on the computed local inhomogeneities of the magnetic field in the surroundings of the needle tips, signal voids in usual gradient echo images were simulated for a prediction of the artifacts that such needles would produce.

The authors suggest that their numerical model might help to instruct a physician in better estimating the needle tip position for different orientations and needle tip shapes. They also suggest that their model might also help to optimize shapes of needle tips and of other parts of MR-compatible instruments and implants with low expense.

The inventor of the present invention has considered the various problems inherent with using known medical instruments with MRI and CT, and believes that the instrument described in his co-pending application, PCT/GB2010/050682, can be rendered more visible not only for use in ultrasonography, but also in a wide range of MRI and CT procedures.

Thus, according to a first aspect of the present invention, there is provided a method of determining the position, in a subject into whom an instrument has been introduced, which comprises an anode and a cathode and in which, in use, current flows between the anode and cathode, liquid inside the body of the subject acting as an electrolyte thereby producing a plurality of gas bubbles which are either directly or indirectly detectable by MRI or CT, the method comprising the steps of: carrying out either a magnetic resonance imaging (MRI) or a computed tomography (CT) procedure; generating, respectively, either an MRI or a CT image; and determining the position of the instrument in the subject based, respectively, on the MRI or the CT image, said gas bubbles rendering said instrument more conspicuous in said image.

In addition, the following disclosure describes, there is provided an instrument, for use in a magnetic resonance imaging (MRI) or computed tomography (CT) procedure, the instrument comprising an anode and a cathode, wherein, in use, current flows between the anode and cathode, thereby producing a plurality of bubbles.

In use, the instrument may be immersed into a subject's body containing firstly fluid, which acts as an electrolyte, and secondly neighbouring cells, tissues or vessels etc., which surround and form around the instrument, also acting as conducting media. As the cells position around the instrument, they form an electrically conducting bridge between the anode and cathode. The cells and the electrolyte complete an electrical circuit with the instrument, and the resulting electrolytic action produces an electric potential between the anode and cathode, such that current flows through the bridging structure.

As ionic current flows through the electrolyte, and electrical current flows through the bridge, gas bubbles are formed at the anode and/or cathode of the needle, i.e. causing effervescence. The inventor has found that making the instrument cathodic with a negative charge causes hydrogen bubbles to form on the instrument. Conversely, if the instrument is made anodic with a positive charge in body tissue, chlorine bubbles will be liberated from its surface owing to the high saline content of body fluids. The fundamental principle of MRI is the detection of mobile protons (i.e. hydrogen ions) in watery tissues. Thus, by virtue of the fact that the gas bubbles (hydrogen or chlorine) generated by the instrument return no signal to an MRI machine detector coil (because they contain no detectable mobile protons), any material which has an electrolysis-induced coating of gas bubbles will appear to have a dark 'aura' or shadow around it on MRI images owing to a process called susceptibility artefact. Similarly, CT scanners display images based on the relative densities of materials. Thus, because the gas bubbles have a very low density, any material which has an electrolysis-induced coating of gas bubbles will also appear to have a dark 'aura' or shadow around it on CT images. The practical implications of this are that the invention allows certain materials, which, owing to their small dimensions or composition are invisible or poorly visible on MRI or CT scanning, to be rendered more conspicuous. Accordingly, the bubbles may be gas bubbles which are either directly or indirectly detectable by MRI or CT.

The injection of air bubbles into a patient through a biopsy needle, so as to allow the biopsy site to be identified later using CT, is known in the art. Specifically, Weerakkody et al., in their paper "The air bubble technique for confirming the location of an image-guided biopsy - a technical note" (British Journal of Neurosurgery, vol. 23, no. 3, June 2009) describe a technique for accurate localisation of a biopsy-site following image-guided biopsy of an intracranial lesion. The authors report that the injection of 0.1 ml of air through a biopsy needle allows the exact location of the biopsy to be visualised on postoperative CT scans performed within 24 hours of the procedure.

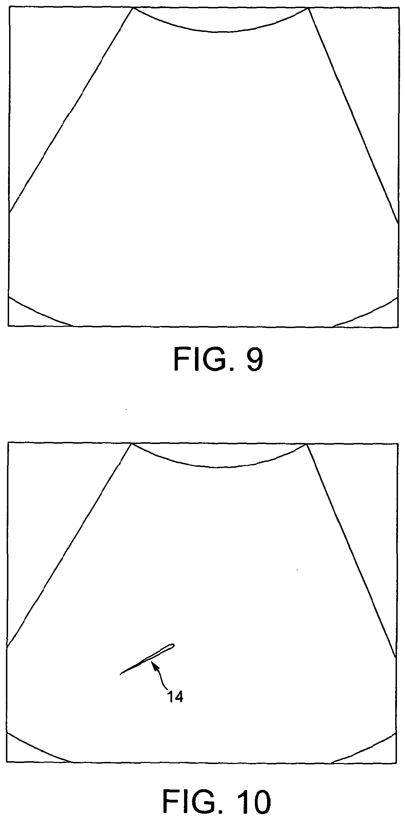

The inventor has found that the instrument of the invention increases the ease with which it can be visualised when inserted inside a patient's body using MRI or CT guidance as shown in Figure 10. Accordingly, the instrument is much easier to visualise and guide during medical procedures. The gas will depend on the composition of the fluid in which the instrument has been inserted. Thus, for example, when inserted into a patient's body, the instrument would electrolyse the bodily fluid (i.e. saline), thereby releasing microscopic bubbles of chlorine (if anodic) and bubbles of hydrogen (if cathodic).

The shape of the instrument used in the first aspect can vary depending on the intended application. For example, in one embodiment, the instrument may be non-cylindrical in shape, and may be less than 2cm in length. The instrument may be any medical device, which is to be inserted into a subject's body, the presence of which is to be identified by MRI or CT. For example, the medical device may be a stent, catheter, port, drug eluting element, electrical stimulator, pacemaker, a breast localization wire or needle, therapeutic or other irradiating element, or other implantable or insertable medical device, the position of which should be monitored during or after a medical procedure.

The instrument may be attached to a medical device made of a non-metallic material which is otherwise invisible or poorly visible by MRI or CT. Examples of such non-metallic materials include, but are not limited to, plastics, ceramics, and other non-metallic composites. The instrument may act as a "beacon" for identifying the position of the medical device under MRI or CT. Similarly, the instrument may be used to allow low density materials, such as, but not limited to, conductive plastic polymers to be rendered visible during biopsies, drainages, and other MRI- or CT-guided procedures.

Thus, the instrument may comprise attachment means by which it may be attached to the medical device. Suitable attachment means may comprise a clip, screw assembly or the like. Alternatively, the instrument may be shaped such that it can be attached to, or inserted into, the medical device.

The instrument may not be percutaneously inserted into a patient. However, the instrument may be a penetrating instrument for percutaneous insertion into a patient. Thus, the instrument used in the first aspect may be a needle. The inventor believes that a needle will be particularly useful for carrying out biopsies and tumour localisations on a patient when guided by MRI or CT. Hence, the needle may be a biopsy or localisation needle. In one embodiment, the needle may be a simple hollow needle comprising a lumen which, after guided insertion into a target tissue (e.g. a tumour), is subjected to a suction force thereby drawing sample cells into the needle lumen for subsequent cytological analysis. This is referred to as fine needle aspiration biopsy (FNAB).

Alternatively, in another embodiment, the needle may be capable of yielding a core of tissue for subsequent histological analysis by virtue of an integral spring-loaded mechanism, for example a stylet. Cutting needle biopsies may have an inner stainless steel solid stylet which has a sharpened end, and an outer stainless steel cutting sheath which may be forced over the stylet by a spring. The biopsy may be trapped in a longitudinal cavity or lumen in the stylet which is situated about 5mm from the sharpened stylet tip. Firstly, in use, the outer needle sheath and stylet may be introduced into a patient together (stylet inside sheath) and directed towards a target site (e.g. a tumour). Secondly, when the target is reached, the inner stylet may be advanced through the target for a distance of 1-2cm. The outer sheath may stay behind at the edge of the target. Tissue naturally "falls" into the longitudinal cavity which has been ground out of the stylet during manufacture. Thirdly, the spring may be activated or 'fired', and the outer cutting sheath snaps forwards over the stylet cutting the sample which now lies free in the longitudinal cavity. The needle and sample may then be removed from the patient.

The calibre of the needle may be between about 8G and 30G. The calibre may be 10G, 12G, 14G, 16G, 17G, 18G, 19G, 20G, 21G, 22G, 23G or 24G. The length of the needle may be between about 1cm and about 50cm, between about 1.5cm and about 30cm, or between about 2cm and about 20cm. Preferably, the outer surface of the needle is substantially smooth to facilitate its insertion into, and its withdrawal out of, a patient's body without the risk of detaching unwanted cells. Since the needle has a smooth external surface, unwanted cells are not picked up as it is inserted and removed from the body, and tumour seeding is therefore less of a problem compared to coated or roughened medical instruments.

The inventor believes that the needle may be used for gaining access to blood vessels either for the purpose of administering intravenous or intra-arterial treatment or for initiating therapeutic procedures via these blood vessels. Cannulas are frequently used in medicine for the administration of injectable medicines and fluids into blood vessels. They comprise an outer plastic sheath to which a syringe or bag of fluid can be attached after placement. A removable central needle allows placement to occur by facilitating puncture of the target and tracking of the plastic cannula over it. Thus, the needle of the invention may be incorporated into a cannula. By increasing the visibility of the needle after bubble generation by an electric current, the ease, speed, safety and tolerance of the cannulation procedure may be optimised using MRI or CT.

Preferably, the anode and the cathode are spaced apart. The instrument may be a bipolar device whereby the anode and cathode are contained within a single assembly whereby the instrument may comprise a separator or insulator material, which is disposed between the anode and cathode. Preferably, the insulator material electrically isolates the anode and cathode from each other, i.e. the insulator material ensures that the anode and cathode are physically separated from each other, thereby avoiding a short circuit and creating a potential difference therebetween, such that, upon insertion in electrolyte, current flows therebetween.

The insulator material may extend around the circumference of the instrument, for example forming an annulus. The insulator material may be tubular-shaped, or in the form of a mesh. The insulator material may be semi-permeable or perforated. The insulator material may comprise a plurality of apertures extending therethough, and through which electrolyte may pass. The insulator material may form a substrate upon which the anode and cathode may be disposed. The insulator material is preferably substantially non-conductive. It should be appreciated that the insulator material is compatible within a biological environment, i.e. physiologically inert and non-toxic to the subject. It is generally preferred to use a polymer material, with at least one or more layer of the polymer being effective without unduly impairing the voltage. Thus, for example, the insulator material may comprise a polymer, for example polyethylene, polytetrafluoroethylene, polysulfide, polymethyl methacrylate, cellulose, alkyl cellulose ethyl cellulose, cellulose acetate, glass, or ceramic.

The anode and/or the cathode may extend around the circumference of the instrument, for example forming an annulus. The anode and/or cathode may be tubular-shaped. The anode and/or the cathode may form a layer or coating on a suitable substrate material, which may be inert. For example, the substrate material may be a polymer, for example polyethylene, polytetrafluoroethylene, polysulfide, polymethyl methacrylate, cellulose, alkyl cellulose ethyl cellulose, cellulose acetate, glass, or ceramic. The substrate may comprise the insulator material. It will be appreciated that the layer of anode/cathode and the interface with the insulator material should form a smooth outer surface to the instrument.

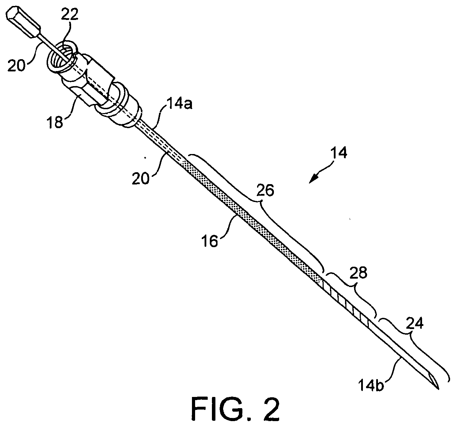

Figures 2 to 8 and Figures 11 and 12 illustrate various embodiments of the instrument (e.g. a needle) which may be used with an MRI or CT apparatus. In each embodiment, the instrument is capable of being observed by MRI or CT due to the materials with which it is made, and because of the gas bubbles generated during electrolysis, which create an aura around it. In one embodiment, the instrument may comprise an anode and cathode of electrically conductive materials of different electrochemical potentials. The anode and cathode are preferably compatible within a biological environment, i.e. physiologically inert and non-toxic to the subject.

The instrument may comprise a metallic material, which generates a low (or no) metallic artefact on MRI or CT. For example, the skilled person will appreciate that different metals have different electrochemical potential values, and lists of these values are readily available in standard chemical textbooks. For example, for the following metals, the electrode potential is given in volts. For calcium: -2.87V, barium: -2.80V, sodium: - 2.71V, magnesium: -2.34V, aluminium: -1.67V, zinc: -0.76V, chromium: -0.74V, iron: - 0.44V, nickel: -0.24V, tin: -0.24V, copper: 0.34V, silver: 0.80V, and gold: 0.80V, and so on.

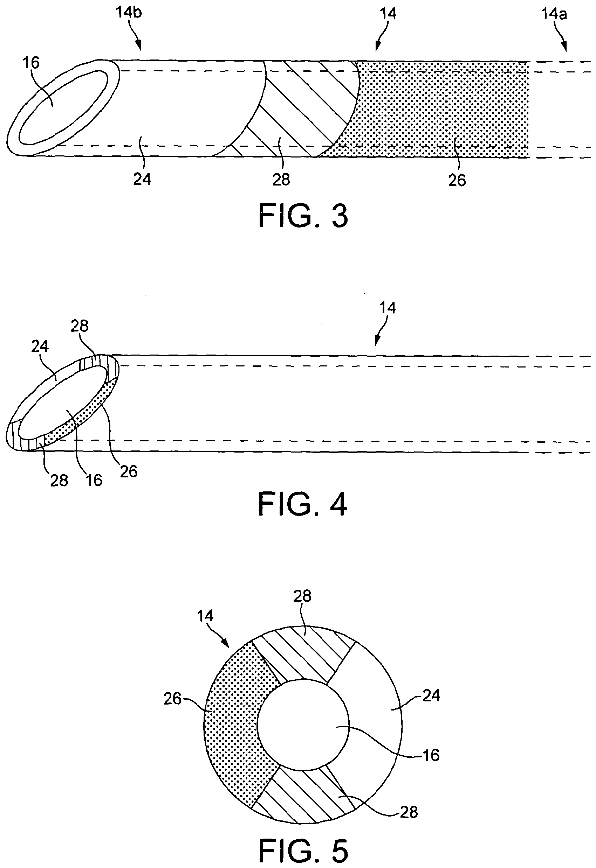

The skilled person will appreciate that pairings of any of these suitable metals (or oxide thereof) may be combined to form the anode and cathode, such that a suitable potential difference is created for electrolysis to occur when the instrument is inserted into a patient's body. For example, the anode may comprise iron, cadmium, aluminium, magnesium, aluminium/magnesium alloy, manganese, or silver. As described in the example, the cathode may comprise tantalum, zinc or nickel. In one preferred embodiment, the anode may comprise iron, and the cathode may comprise tantalum. In another preferred embodiment, the anode may comprise cadmium, and the cathode may comprise nickel. In another preferred embodiment, the anode may comprise zinc, and the cathode may comprise silver oxide. In another preferred embodiment, the anode may comprise zinc, and the cathode may comprise manganese dioxide/carbon. In one embodiment, the anode and cathode may form spaced apart sections which are disposed along a longitudinal axis of the needle, which sections are preferably separated by a section of insulator material. This embodiment is illustrated in Figures 2 and 3.

For example, the anode may be disposed at a distal end of the instrument, and the cathode may be disposed at a proximal end of the instrument, preferably with a section of insulator material being disposed therebetween. Alternatively, the anode may be disposed at a proximal end of the instrument, and the cathode may be disposed at a distal end of the instrument, preferably with the insulator material being disposed therebetween. Provided that the anode and cathode are separated by the insulator, either arrangement will result in a potential difference being created, such that a current flows therebetween upon insertion into an electrolyte (i.e. in a subject's body). The inventor believes that the provision of the insulator material prevents the electrical circuit formed upon insertion of the instrument into the electrolyte from 'shorting', which would other wise prevent electrolysis from occurring.

The length of the sections of anode, cathode and insulator material are such that a current of sufficient voltage is created to produce the gas bubbles. For example, the sections of anode, cathode and insulator material may be approximately equal in length. The anode and/or the cathode may be provided as a coating on a suitable substrate material, forming a smooth outer surface. The substrate is preferably inert (such as ceramic), and may comprise the insulator material. Hence, in some embodiments, the anode and cathode may adhere to the inert substrate, leaving a space therebetween, which acts as the insulator section.

In another embodiment, the anode and cathode may form spaced apart sections which are disposed around the circumference of the instrument, preferably separated by a section of insulator material. This embodiment is shown in Figures 4 and 5. For example, the anode section may be disposed on one side of the circumference of the instrument, and the cathode may be disposed on a mutually opposing side of the instrument's circumference. Preferably, the instrument comprises at least two sections of insulator material disposed in between the anode and cathode around the circumference of the instrument. As with the embodiment discussed above, the anode and/or the cathode may also be provided as a coating on a suitable substrate material, which is preferably inert. The substrate may comprise the insulator material itself, such that, in some embodiments, the anode and cathode may adhere to the substrate material, leaving a space (and preferably two spaces) therebetween, which acts as the insulator sections. It is preferred that the coatings on the substrate form a smooth surface over the external surface of the instrument.

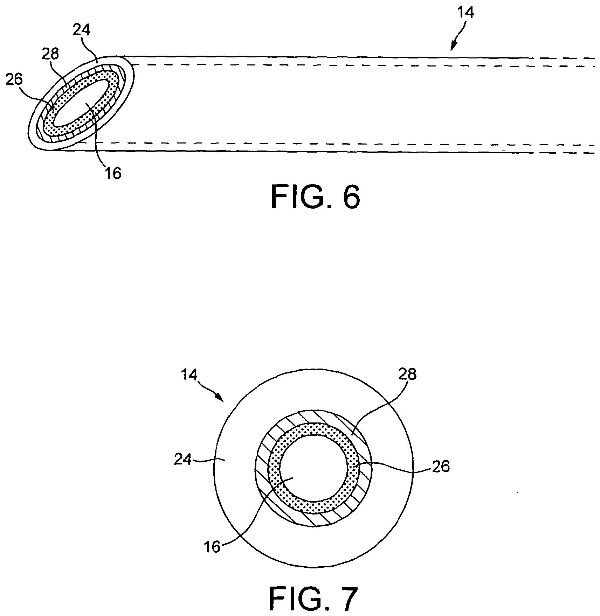

In still another embodiment, the anode and cathode are arranged as radially spaced apart, concentric elements, preferably separated by an element comprising insulator material. This embodiment is depicted in Figures 6 and 7. For example, the instrument may comprise an inner cathode, and an outer anode and preferably an intermediate insulator material section, which separates the anode and cathode. Alternatively, the instrument may comprise an inner anode, and an outer cathode, and preferably an intermediate insulator material section. It is believed to be important that the insulator allows electrolyte to flow between the anode and cathode. Thus, in this embodiment, the insulator may comprise a plurality of apertures extending therethrough, through which electrolyte may flow. For example, the insulator may comprise a mesh or a semi-permeable membrane.

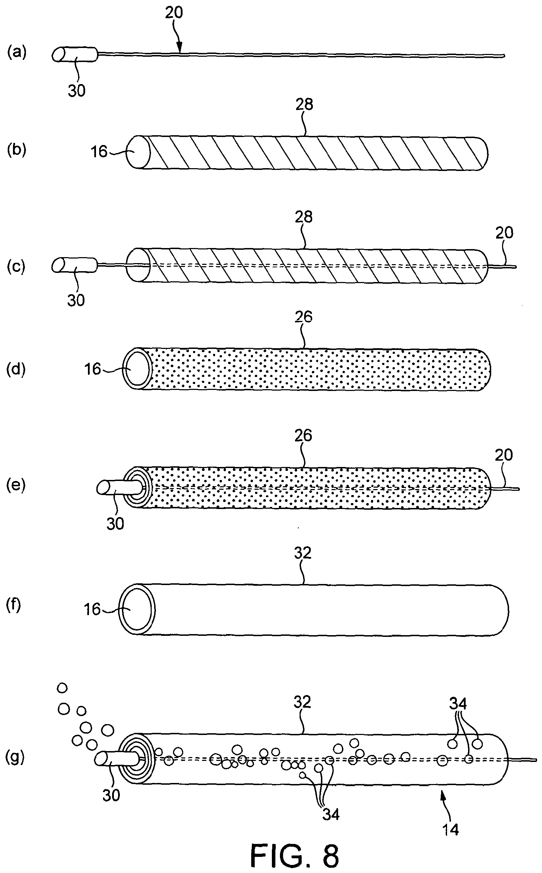

In yet another embodiment, which is shown in Figure 8, the anode or cathode may comprise an outer hollow tube having a lumen extending therethrough, and the reciprocal electrode of opposite charge may comprise a stylet slidably mounted through the lumen. The stylet may comprise cutting means at a distal end thereof. The cutting means may comprise a sharpened cutting edge capable of cutting into a target tissue within the subject's body. The needle may comprise an insulator material disposed between the anode and the cathode. The insulator material may be tubular-shaped. The instrument may comprise an outer coating of semi-permeable material in the form of a cylinder having a lumen extending therethrough. The coating may be disposed around the cathode, and arranged, in use, to trap bubbles of gas therebetween.

In use, this embodiment of the instrument may be inserted into a tissue of a subject, such as liver, in order to take a biopsy. Upon insertion into the tissue, the potential difference between the stylet acting as the anode, and the cathode cylinder, results in electrolysis taking place such that electrical current flows through the tissue, and ionic current flows through the fluid in the tissue. This results in the formation of bubbles of gas, many of which are trapped between the cathode and the outer semi-permeable coating. The bubbles ensure that the position of the instrument is readily visible inside the patient by MRI or CT. The operator may move the instrument inside the patient, continuously checking its position on the monitor, until it reaches a target site, at which point, the operator may activate the stylet such that the cutting means cuts into, and removes, a biopsy. The cutting means may be withdrawn from the target site, thereby retaining the biopsy sample at the distal end of the instrument. The instrument may then be removed from the patient taking the biopsy with it.

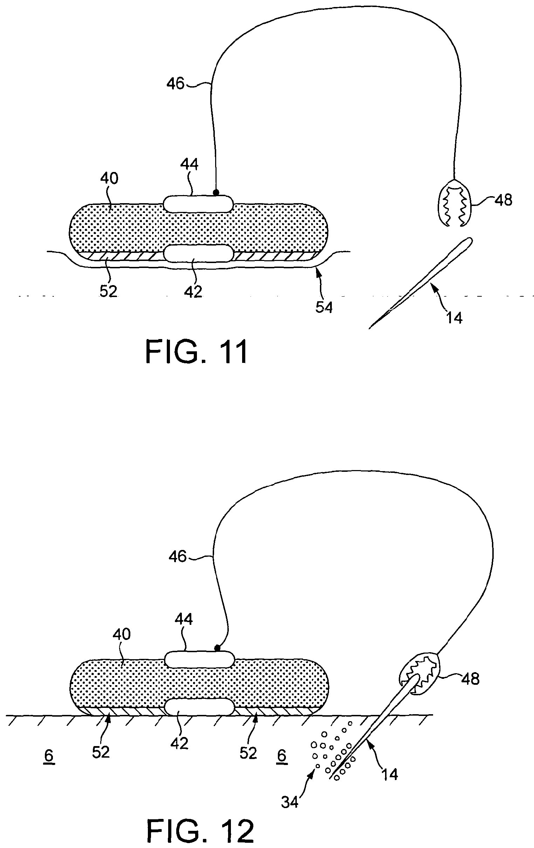

In a further embodiment, which is shown in Figures 11 and 12, the instrument may comprise a power source, which generates electrical current which flows between the anode and cathode. It will be appreciated that MRI and CT apparatuses involve the use of large magnets for generating strong magnetic fields. Thus, preferably, the power source is compatible with MRI or CT apparatus, or contained within a housing which is MRI or CT compatible. The power source therefore may be substantially non-magnetic. For example, this may be achieved by using non-ferrous materials.

For example, a suitable non-magnetic MRI compatible lithium battery may be used, which may be supplied by an MRI or CT equipment manufacturer. One example of such a manufacturer is MRIequip, Nisswa, USA, who produce an MRI compatible lithium battery for a laryngoscope. The power source may be a lithium ion bipolar non-magnetic battery commonly used in the space program and other MRI applications. Furthermore, US 2010191069 discloses a battery system which is MRI compatible, which may be used in accordance with the present invention. Also, WO 2006099011 discloses a wireless in-bore sensor for MRI, which is battery operated, and which may also be used as the power source described herein.

In another embodiment, the power source may be disposed outside the MRI or CT apparatus, and connected to the instrument inside the MRI or CT apparatus with MRI compatible material.

Hence, in this embodiment, the anode and cathode may comprise the same or similar material having the same or substantially similar electrochemical potentials. For example, the anode and cathode may both comprise aluminium, iron, iron alloy, silver, stainless steel, titanium or other alloys. In this embodiment, in contrast to the other embodiments, electrical current is provided by means of the power source, which provides the electromotive potential so that an anode and cathode are created, such that tiny bubbles of gas are produced, which increase the conspicuity of the instrument by MRI or CT. The power source may be integral with the instrument, or it may be attached thereto via one or more electrically conductive wires. For example, the power source may comprise a battery.

The power source, and preferably a cathode thereof, may be attachable to the instrument by at least one electrically conductive wire, and preferably via suitable attachment means, for example a crocodile clip or the like. In one embodiment, the power source may be embedded in an electrically conducting adhesive substance, for attachment to a subject on which MRI or CT is to be carried out. In another embodiment, the power source may comprise a region of electrically conducting adhesive substance extending along at least one surface thereof, preferably at least adjacent an anode of the power source. The power source may comprise a removable backing layer extending substantially over the adhesive substance, which layer prevents the adhesive from sticking to unwanted surfaces prior to use.

In use, an operator may remove the backing layer from the conducting substance, which may then be placed on a subject, preferably at a suitable position to coincide with the inserted instrument. When the power source is in position, the anode contacts the surface of the subject, thereby acting as a reference electrode (i.e. anode). The operator may connect the cathode of the power source to the instrument, which thereby acts as the cathode. Upon insertion of the instrument into the subject, an electrical circuit is created therein, such that electrolysis occurs, thereby forming gas bubbles on the surface of the instrument. The operator may then carry out MRI or CT on the subject. As the bubbles do not contain any mobile protons, a dark or black shadow is formed around the instrument on MRI images. Also, since the bubbles have a very low density, a dark or black shadow is formed around the instrument on CT images. Thus, in each case, the operator can visualise the position of the instrument inside the subject.

The power source may comprise a battery, for example a 1.5V, 3V, 4.5V, 6V, 7.5V or 9V battery. In another embodiment, the power source may be incorporated into a non-adhesive
housing which completes an electrical circuit by apposition (upon contacting) to the skin surface using an electrically conducting material, such as gel.

Preferably, the instrument comprises (or consists entirely of) a non-metallic material. Examples of suitable non-metallic materials may include, but are not limited to, glass, plastics, ceramics, and other non-metallic composites. The inventor believes that the non-metallic nature of the instrument for use in MRI or CT procedures is an important aspect of the invention.

In addition, the following disclosure describes a non-metallic MRI or CT instrument comprising an anode and a cathode, wherein, in use, current flows between the anode and cathode, thereby producing a plurality of bubbles.

The instrument may be a penetrating instrument which is inserted percutaneously into a patient, and may be a needle. The needle may be a biopsy or a localisation needle. The instrument may comprise or be made substantially entirely of glass, plastic, ceramic, or some other a non-metallic composite, which is rendered more visible under MRI or CT. Preferably, the instrument is capable of conducting electrical current so that the bubbles are produced when the current flows between the anode and cathode.

The inventor believes that the instrument described herein may be used in various methods involving MRI or CT procedures.

The following disclosure therefore describes the use of an instrument in a magnetic resonance imaging (MRI) or computed tomography (CT) procedure, the instrument comprising an anode and a cathode, wherein, in use, current flows between the anode and cathode, thereby producing a plurality of bubbles.

Further, the following disclosure describes a method of carrying out a magnetic resonance imaging (MRI) procedure on a subject, the method comprising the steps of:
(a) introducing, into a subject, an instrument comprising an anode and a cathode, wherein, in use, current flows between the anode and cathode, thereby producing a plurality of bubbles; and
(b) carrying out MRI on the subject.

Still further, the following disclosure describes a method of carrying out a computed tomography (CT) procedure on a subject, the method comprising the steps of:
(a) introducing, into a subject, an instrument comprising an anode and a cathode, wherein, in use, current flows between the anode and cathode, thereby producing a plurality of bubbles; and
(b) carrying out CT on the subject.

The methods may comprise inserting the instrument into the subject, which contains fluid that acts as an electrolyte. The methods may comprise forming a conducting bridge comprising subject's cells/cellular and extracellular fluid, between the anode and cathode. The methods may comprise creating a current which flows between the anode and cathode. The methods may comprise creating an electrical current in the conducting bridge. Preferably, the methods comprise creating an ionic current in the electrolyte. The methods may comprise creating gas bubbles at the cathode and/or anode.

The instrument used in the methods may comprise or consists of glass, plastic, ceramic, or some other a non-metallic composite. Preferably, the instrument is capable of conducting electrical current. The instrument may be a needle, such as a biopsy or a localisation needle.

The subject may be a vertebrate, mammal, or domestic animal, and is preferably a human being. However, the methods can be applied to enhance the visibility to MRI or CT of any electrically conductive object or medium immersed in an electrically conductive solution.

The instrument and methods according to the invention may be used for carrying out a wide range of medical applications, for example for obtaining a small cell sample from a tumour, or the like, i.e. a biopsy. The instruments and methods may also be used for other medical applications in which a needle is inserted into a subject, for example inserting a catheter, insertion of a central line, in achieving anaesthesia (e.g. regional or local anaesthetic), in keyhole surgery and in breast tumour localisation and marker wire placement. The instruments, apparatus and methods may be used in cosmetics, such as injection of collagen, or botulinum toxin into a desired location while avoiding non-target structures, e.g. pleura, nerves and blood vessels, or in liposuction, in which the ability to retrace where a suction needle has been placed previously is thought to be particularly useful for removal of fat. MRI or CT guidance is also used for sclerotherapy, i.e. during treatment for varicose veins or vascular malformations.

The instruments and methods of the invention may also be used during interventional procedures, such as nephrostomy, percutaneous biliary drainage, percutaneous gastrostomy insertion, abscess drainage, drainage of ascites, insertion of a catheter into the peritoneal cavity, artery or vein cannulation or catheterisation. The instruments and methods may also be used for atherectomy (which feeds a catheter through the arm or groin to central blood vessels), drainage and tissue ablation.

The inventor has found that the instrument is particularly suitable for initiating drainage procedures such as nephrostomy and biliary drainage under MRI or CT guidance. These procedures are often followed by stent insertion. In these situations, the dilated intrarenal urinary collecting system, or intrahepatic biliary tree, may be accessed with a fine needle directed towards the target using MRI or CT guidance. When the needle tip has entered the dilated system, using a Seldinger technique of initial wire insertion through the needle followed by needle withdrawal and subsequent insertion of catheters over the wire, drainage and stent insertion can be facilitated.

The inventor has also found that the administration of anaesthetic agents can be facilitated by guiding the needle under MRI or CT towards the location of a nerve. On reaching the target, the anaesthetic (e.g. a local anaesthetic) can be infiltrated around the nerve resulting in the achievement of local or regional anaesthetic. Examples of such regional nerve 'blocks' include, but are not limited to, femoral, brachial, radial, axillary, and intercostal nerve blocks. Local anaesthesia can be achieved by guiding the needle into any target tissue using a percutaneous route. Examples of local anaesthetic include, but are not limited to perivascular infiltration of anaesthetic around any target blood vessel, e.g. femoral, brachial, radial, axillary arteries or veins, jugular veins etc.; infiltration of local anaesthetic around a biopsy site such as an abdominal, thoracic, pelvic, cervical, cranial, peripheral or other subcutaneous organ or lymph node.

All of the features described herein (including any accompanying claims, abstract and drawings), and/or all of the steps of any method or process so disclosed, may be combined with any of the above aspects in any combination, except combinations where at least some of such features and/or steps are mutually exclusive.

For a better understanding of the invention, and to show how embodiments of the same may be carried into effect, reference will now be made, by way of example, to the accompanying Figures, in which:-
Figure 1 shows a schematic side view of an MRI apparatus, including an instrument according to the invention, being used on a patient;
Figure 2 shows a perspective view of a first embodiment of the needle shown in Figure 1;
Figure 3 shows an enlarged cross-sectional side view of the first embodiment of the needle shown in Figure 2;
Figure 4 shows an enlarged cross-sectional side view of a second embodiment of the needle;
Figure 5 shows an end view of the second embodiment of the needle shown in Figure 4;
Figure 6 shows an enlarged cross-sectional side view of a third embodiment of the needle;
Figure 7 shows an end view of the third embodiment of the needle shown in Figure 6;
Figures 8(a) to (g) show cross-sectional side views of a fourth embodiment of the needle;
Figure 9 is a representation of an MRI image showing a prior art 22G needle positioned about 10cm inside a body, which has undergone MRI;
Figure 10 is a representation of an MRI image showing a 22G needle according to the invention positioned about 10cm inside a body, which has undergone MRI;
Figure 11 is a schematic side view of a fifth embodiment of the needle of the invention; and
Figure 12 is a schematic side view of the needle shown in Figure 11 in use.

### Examples

### Example 1

Referring to Figure 1, there is shown an MRI apparatus 2 according to the invention. MRI measures the signal return from mobile protons, which are abundantly present in the human body. Protons (hydrogen nuclei) have diamagnetic properties with a significant magnetic moment, which allows their signal to be used to generate images. The MRI apparatus 2 may be used for carrying out a biopsy on a patient 6, for example for obtaining a small cell sample from a tumour, or the like. However, the apparatus 2 may also be used for a wide range of other medical applications in which a needle is inserted into the body of a patient 6 in an MRI or CT machine, for example: (i) attachment of a cannula to a patient, (ii) in anaesthesia (i.e. administration of an anaesthetic to a patient, such as an epidural), (iii) insertion of a central line (i.e. a tube inserted directly into a major blood vessel, such as a vein or an artery near the heart), (iv) keyhole surgery, (v) in cosmetics, and (vi) in breast tumour localisation and marker wire placement.

The apparatus 2 shown in Figure 1 includes an instrument, such as a needle 14, which has a sharp tip, which, in use, pierces the skin of the patient 6 under investigation at position 14b. Various embodiments of the needle 14 are described in detail below, and are illustrated in Figures 2 to 8. The MRI apparatus 2 shown in Figure 1 includes large magnets 4, which produce a magnetic field in the direction shown by arrow 'X'. The patient is placed in a strong electromagnetic field 'X' of 0.3 to 3 Teslas (specialized MRIs use up to 7 T). The billions of protons in the body align themselves parallel with the magnetic field 'X', either in the same direction or opposite to the direction of the field. At the level ("slice") where it is desired to "take a picture", a short, and powerful radio signal is sent through the body, perpendicular to the main magnetic field, as represented by arrows 'Y'. The protons, which can vibrate with the same frequency as the impinging radio wave, become "excited" (i.e. they will be raised to a higher state of energy), and start to resonate with the frequency of the exciting wave.

When the radio signal 'Y' is turned off, the protons will, after a period of time, return to their original energy state. The excitation energy, which they had gained, is then released in the form of radio waves, presented by arrow 'Z', which are detected by sensors 6. The strength of this detected signal is proportional to the proton density (PD). The time it takes for the excited H-nuclei to return to their original energy level, the relaxation time, is also measured and analyzed by a computer. There are two types of emitted radio waves, with relaxation times T1 and T2. The former relies on the direct surrounding of the spinning proton and the latter on the mobility of the proton. One of the three (density, T1 or T2) can be used for the image, but a combination is also possible. For the contrast in the image and so for the interpretation of the image, information about what is used is needed. To make an image, one must know where the emitted RF signal comes from, and so one needs 3D information. The RF coils or antennae (not shown) are not direction sensitive, and so do not provide this information. Therefore, the position of the RF signal is provided by the x, y and z gradient coils (not shown).

MRI may be enhanced by the use of contrast agents containing elements of a higher atomic number than the surrounding water-like tissues. Contrast agents for MRI are those which are strongly paramagnetic, such as gadolinium. Intravenous administration of such agents prior to imaging is known as contrast-enhanced MRI.

Referring to Figure 9, there is shown an MRI image of a prior art 22G needle inserted into a patient. As shown in Figure 9, it is not possible to see the prior art needle satisfactorily under MRI, because the metal substrate causes susceptibility artefact resulting in a 'blooming' of the signal. The resultant image shows the needle to be much wider than it actually is, and in the process may obscure the target tissue, , and so it is very difficult to use in most MRI procedures. However, with reference to Figure 10, since the needle 14 of the invention is preferably non-metallic, it does not cause 'blooming' of the signal or obscuration of the target tissue. The non-metallic material, which would normally be poorly visible or invisible on MRI, is attached to the invention generating hydrogen gas bubbles. These do not contain any mobile protons, and so the needle 14 is surrounded by a dark region or aura which renders the needle 14 highly conspicuous under MRI. As shown in Figure 10, the position of the needle 14 of the invention inside the patient 6 can be clearly seen in an MRI image on a monitor (not shown). Although not shown, the inventor believes that the needle 14 would also be rendered conspicuous under a CT scan because the gas bubbles have a very low density compared to the surrounding tissue and the needle 14 itself. Thus, again, the bubbles would create a dark aura around the needle 14, which would therefore be highly visible under CT.

In each embodiment illustrated in Figures 2 to 8, and 11 to 12, the needle 14 of the invention is rendered visible in MRI or CT. In most embodiments, the needle 14 comprises at least two metals having different electrochemical potentials, such that one of the metals forms an anode 24 and the other forms a cathode 26. The two metals forming the anode 24 and the cathode 26 are physically separated and therefore electrically isolated from each other by an insulator 28, which thereby creates a potential difference therebetween. Upon insertion of the needle 14 into a patient's body, liquid inside the body acts as an electrolyte, and conducting cells adjacent the needle 14 forms a conducting bridge between the anode 24 and cathode 26, such that current flows between the anode 24 and cathode 26. Ionic current is thought to flow through the electrolyte, whereas electrical current is thought to flow through the conducting bridge formed between the anode and cathode by neighbouring cells. The principle of the needle 14 therefore is that it acts as an electricity source capable of generating a voltage (of about 15mV) which is sufficient to electrolyse the liquid medium in the body (i.e. the electrolyte) surrounding the needle 14. For example, when inserted into subcutaneous fat and tissue of a patients body 6, the needle 14 electrolyses saline releasing chlorine gas at the anode 24, and hydrogen gas at the cathode 26. The gases are released as low density microscopic bubbles, which do not contain any mobile hydrogen ions or protons and so do not create a signal which can be detected by MRI or CT. They therefore render the needle 14, from which the gas is produced, highly visible using MRI or CT using apparatus 2.

The needle 14 therefore acts as a battery, and so at least in some embodiments of the invention, does not require the use of an external electrical source. The materials and construction of the needle 14 (i.e. the anode 24, cathode 26, and insulator 28) must meet a number of criteria. The primary criteria are that: (i) the materials forming the anode 24 and cathode 26 have different electrochemical potentials; and (ii) the insulator 28 electrically isolates the anode 24 and cathode 26 from each other, to create the potential difference. When a needle 14 meeting these criteria is positioned inside the patient's body 6, it is effectively being immersed in an electrolytic fluid. For example, if the needle 14 is positioned in the body 6, the saline surrounding the organs constitutes the electrolytic liquid, and the organ in which the needle 14 has been inserted forms a conducting bridge between the anode 24 and cathode 26. If the needle 14 is positioned in a liver bile duct, for example if carrying out a biopsy from the bile duct, the bile itself constitutes the electrolytic liquid. If the needle 14 is inserted into the vascular network, for example when inserting a mainline or even a simple cannula, blood constitutes the electrolytic liquid, and so on. As the needle 14 is inserted into the patient's body, an electrical circuit is formed between the anode and cathode by the conducting bridge and the electrolyte. An electric potential difference between the anode 24 and cathode 26 is formed, such that a current flows therebetween.

Other material selection and construction criteria depend upon the specific application intended for the needle 14. For example, selected materials should be compatible within a biological environment, i.e. each material should be physiologically inert, neutral and non-toxic the patient 6. Electrolysis occurring inside the patient's body will erode the anode 24 and, provided that the anode, cathode and insulator materials are all physiologically neutral, any by-products of electrolysis will not have an adverse impact on the patient 6.

The inventor has found various combinations of materials that may be used for the anode 24 and cathode 26 of the needle 14 of the invention. One combination is tantalum (as the cathode) with iron (as the anode). Another combination is nickel (as cathode) and cadmium (as anode). Aluminium-magnesium alloy or silver are further suitable anode 24 materials, but the generated output voltage with a silver anode may be less than with an iron anode. The intermediate insulator 28 can be formed of any physiologically inert or neutral non-conductive material, for example a polymer, such as polyethylene, or the like. The purpose of the insulator 28 is to electrically isolate the anode 24 from the cathode 26 so that a potential difference therebetween is created, such that tiny gas bubbles are formed, thereby increasing the conspicuity of the needle 14 with MRI or CT.

Each of the various embodiments of the needle 14 according to the invention which can be used with the apparatus 2 shown in Figure 1 will now be described in detail. Referring first to Figures 2 and 3, there are shown views of a first embodiment of the increased visibility needle 14 for use in percutaneous procedures under MRI or CT guidance. The needle 14 comprises an elongate shaft having proximal and distal ends 14a,14b, the latter of which is sharply pointed for piercing the skin of the patient 6. The needle material is made of an electrically conductive substance which does not register large artefact on MRI. Another embodiment is that the needle material is made of an electrically conductive substance which does not register large artefact on CT. The calibre of the needle 14 is between about 12G and 25G, and its length is between about 2cm and 20cm. The outer surface of the needle 14 is substantially smooth to facilitate its insertion into, and its withdrawal out of, a patient's body without the risk of detaching unwanted cells. A smooth, internal, cylindrical lumen 16 extends between the ends 14a,14b along which fluid or bodily materials, such as a biopsy cell sample, may pass. In the first embodiment shown in Figure 2, the needle 14 includes a mounting 18 generally made of a plastic material which surrounds, and is secured to, the proximal end 14a. An optional stylet 20 is slidably and removably disposed within the lumen 16 of the needle 14, and substantially blocks the distal end 14b, and, as described hereinafter, helps form a cutting edge for use during the insertion process. When removed from the needle 14, the stylet 20 exposes a threaded or ribbed portion 22 within mounting 18 onto which a hypodermic syringe may be removably secured and used in a manner well-known in the art.

The needle 14 shown in Figures 2 and 3 is composed of three distinct sections disposed along a longitudinal axis of the needle 14, i.e. (i) the anode section 24 made of iron, which forms the distal end of the needle 14b, (ii) the cathode section 26 made of tantalum, which forms the proximal end of the needle 14a, and (iii) the intermediate insulator section 28 made of polyethylene, which physically separates the anode 24 and cathode 26, thereby creating a potential difference. Although not shown in the Figures, it should be appreciated that either the anode 24 or the cathode 26 can be disposed at either the distal end or the proximal end 14a of the needle 14b. Provided that the anode 24 and cathode 26 are separated by the insulator 28, either arrangement will result in a potential difference being created therebetween.

Once inserted into a patient's body, the needle 14 generates an electrolytic voltage of about 15mV by electrolysis. As electrolysis occurs, microscopic bubbles of gas are produced on the surface of the anode 24 and cathode 26, the bubbles sticking to the surface of the needle 14 by surface tension. As with all embodiments of the needle 14, the type of gas contained within the bubbles is determined by the composition of the electrolyte, which in turn depends upon the position of the needle 14 inside the patient's body. The bubbles do not contain any mobile protons (H ions) and have a very low density compared to the needle 14 and surrounding tissues. They therefore surround the needle 14 producing a dark region, which is clearly detectable by MRI or CT using apparatus 2. Once the needle 14 has been carefully guided into position, the operator then either takes a biopsy from, or delivers a medicine or the like to, the target site. The needle 14 may then be carefully removed from the target site in the patient 6, and, if necessary, re-inserted at a different position or angle. Due to the metallic composition of the needle 14, bubbles continue to be generated, thereby enabling the operator to view the needle *in situ* as many times as required.

Referring next to Figures 4 and 5, there are shown views of a second embodiment of the needle 14 for use in percutaneous procedures under MRI or CT. As shown most clearly in Figure 5, the needle 14 is composed of four distinct sections, which are disposed around the circumference of the needle 14, i.e. an anode section 24 made of cadmium on one side of the circumference of the needle 14, a cathode section 26 made of nickel on the opposite side of the needle's circumference, and two sections of insulator material 28 made of ceramic, which physically separates the anode 24 and cathode 26 sections, thereby creating the potential difference. The lumen 16 is shown in the centre of the needle 14. It will be appreciated that there is no physical contact between the anode 24 and cathode 26 due to the insulator 28, and because of the lumen 16.

Upon insertion of the needle 14 into the body of a patient, the needle 14 automatically generates an electrolytic voltage between the spaced-apart anode 24 and cathode 26, which in turn results in the formation of tiny bubbles of gas. As discussed above, the presence of the bubbles on the surface of the needle 14 improves its conspicuity to MRI or CT, as they do not return a signal, and greatly facilitates an operator in guiding the needle 14 through the patient's body.

Referring to Figures 6 and 7, there are shown views of a third embodiment of the needle 14 according to the invention. As shown most clearly in Figure 7, the needle 14 is arranged with a series of nested or concentric elements, i.e. an inner cathode 26 (tantalum), an outer anode 24 (silver), and an intermediate insulator 28, which separates the anode 24 and cathode 26, which sections collectively form a three-layer cylindrical structure. The lumen 16 is shown in the centre of the needle 14. Although not shown in the Figures, it will be appreciated that either the anode 24 or the cathode 26 can form the inner of outer layer of the needle 14. Provided that the anode 24 and cathode 26 are separated by the insulator 28 layer, either arrangement will result in a potential difference being created. In this embodiment, the insulator 28 is provided as a polymer mesh, or a semi-permeable membrane, having numerous apertures extending therethrough, through which electrolyte can flow. Thus, the insulator mesh 28 prevents the anode 24 and cathode 26 from contacting each other, as otherwise prevent a potential difference from being created. However, the mesh 28 also ensures that electrolyte can flow between the anode 24 and cathode 26 so that electrolysis can occur. Once inserted into a patient's body, the needle 14 automatically generates an electrolytic voltage, which results in the production of tiny gas bubbles, which improve the visibility of the needle 14 to MRI or CT guidance using apparatus 2. As with other embodiments of the needle 14, the operator can guide the needle 14 through the body of the patient by viewing its position on the monitor 12.

Referring to Figure 8(g), there is shown a fourth embodiment of the needle 14 according to the invention, and Figures 8(a) to (f) show the various components which collectively make up the needle 14. Figure 8(a) shows an elongate, inner stylet 20, at the distal end of which is located a sharp cutting edge 30. The stylet 20 is made of iron and acts as the anode of the needle 14. Figure 8(b) shows the polyethylene insulator 28 which is formed into a cylindrical mesh having lumen 16 extending therethrough. As shown in Figure 8(c), the stylet 20 is disposed within, and extends through, the lumen 16 of the insulator mesh 28. The mesh 28 has a plurality of apertures extending therethrough, through which electrolyte may flow.

Figure 8(d) illustrates the cathode made of tantalum, which is cylindrical in shape having lumen 16 extending therethrough. As shown in Figure 8(e), the arrangement of the stylet 20 disposed within the insulator mesh 28 is in turn disposed within the cathode cylinder 26. Thus, the insulator mesh 28 prevents the cathode 26 contacting the anode stylet 20, but ensures that electrolyte can flow therethrough between the anode 24 and cathode 26. Finally, Figure 8(f) illustrates a semi-permeable coating, which in one embodiment, is made of plastic in the form of a cylinder 32 having a lumen 16 extending therethrough. As shown in Figure 8(g), the arrangement depicted in Figure 8(e) is disposed inside the semi-permeable coating 32 shown in Figure 8(f), thereby forming the complete needle 14.

In use, the needle shown in Figure 8(g) is inserted into a tissue of a patient 14, for example a lung, in order to take a biopsy. Upon insertion into the body 6, the potential difference between the stylet 20 acting as the anode, and the cathode cylinder 26 results in a 15mV current being created. The electrical current causes electrolysis inside the lung, resulting in the formation of tiny bubbles 34 of gas (chlorine and hydrogen), the majority of which are trapped between the cathode 26 and the semi-permeable coating 32, as shown in Figure 8(g). The bubbles 34 do not contain any mobile protons and have a low density, and so do not return a signal under MRI or CT, such that the operator can easily see the position of the needle 14 inside the patient by a dark region surrounding the needle 14. The operator then moves the needle 14 inside the patient, continuously checking its position on the monitor 12, until it reaches the target site, i.e. the tumour, at which point, the operator activates the stylet 20 such that the cutting edge 30 cuts into, and removes, the biopsy. The cutting edge 30 is withdrawn from the target site, and insodoing, retains the biopsy sample at the distal end 14b of the needle 14. The needle 14 is then carefully removed from the patient taking the biopsy with it. Throughout the whole procedure, the operator is able to clearly see the position of the needle 14 inside the patient's body due to the production of microscopic gas bubbles which surround the needle 14 and which are not detectable by MRI or CT producing a dark region therearound. Therefore, the needle 14 can be accurately manoeuvred through the body 6.

In a modification of this embodiment, a battery 40 can be either attached to, or integrated into, the shaft of the needle 14. The anode of the battery 40 can be electrically connected to the stylet 20, and the cathode of the battery 40 can be connected to the cylindrical cathode made of tantalum 16, or *vice versa.* The inventor has found that a tiny battery 40 normally used in a hearing aid or mobile phone is suitable for integrating into the body of the needle 14, and generates sufficient current to cause electrolysis and therefore form gas bubbles. The inventor has used a battery 40 as described in US 2010191069, which is MRI compatible. Alternatively, the battery can be a normal magnetic battery which is contained within an MRI compatible housing, or it may be positioned outside of the MRI apparatus 2, but connected to the needle 14 by MRI compatible wires.

Referring to Figures 11 and 12, a fifth embodiment of the needle 14 is illustrated, which involves a needle 14 in which the same metals (having the same or similar electrochemical potential) are used as the anode 24 and cathode 26. For example, the anode 24 and cathode 26 can both be made of stainless steel. However, in this embodiment, in contrast to the other embodiments, electrical current is provided by means of a separate power source, such as a battery 40. The battery 40 provides the electromotive potential so that an anode 24 and cathode 26 are created, such that tiny bubbles 34 of gas are produced, which increase the conspicuity of the needle 11 by MRI or CT, as they do not return a signal due to lacking protons and have a low density.

As shown in Figure 11, the battery 40 (by way of example only: 1.5V, 3V, 4.5V, 6V, 9V or 12 Volt) has its own anode 42 and cathode 44. The cathode 44 is attached to a connector 48, such as a crocodile clip, by an electric wire or cable 46. The connector 48 is used to connect the battery 40 to the conductive barrel of any chosen needle 50, and the length of the wire 46 allows sufficient movement to deploy the needle 50 into a subject's body 6, as shown in Figure 12. The battery 40 can be either embedded in an adhesive gel-like substance 52, or have a thin layer of a conducting, but sticky gel-like substance 52 extending along one surface thereof, where the anode 42 is disposed. A peelable layer of material 54 is provided as a backing sheet over the gel-like substance 52 in order to prevent it from sticking to unwanted surfaces before it is used.

Referring to Figure 12, the fifth embodiment of the needle 14 is shown in use. The operator first unpeels the backing sheet 54 away from the conducting sticky substance 52 of the battery 40, which is then placed on a subject's body 6 at a suitable position to coincide with needle 14 placement. When the battery 40 is in position, the anode 42 contacts the surface of the subject's body 6, thereby acting as a reference electrode (anode). Then, the operator connects the crocodile clip 48 to the needle 14, which acts as the cathode. Upon insertion of the needle 14 into the subject 6, an electrical circuit is created in the body 6, such that electrolysis occurs, thereby forming hydrogen bubbles 34 on the surface of the needle 14 when the needle is charged as a cathode. If the needle is charged as an anode, chlorine bubbles will be formed and these may also improve the conspicuity to MRI or CT.

The operator then carries out MRI or CT on the subject 6. The gas bubbles 34 do not contain any mobile protons and so do not return a signal to an MRI detector. Similarly, the bubbles 34 have a low density compared to the needle 14 and the surrounding tissues, and so again return no signal to a CT detector. Accordingly, the operator can visualise the position of the needle 14 inside the patient 6 using either CT or MRI. The needle 14 can be moved inside the patient 6, and its position can be seen on an MRI monitor, until it reaches the target site. It will be appreciated that both monopolar (i.e. one electrode) or bipolar (i.e. two electrodes) embodiments may be used.

In summary, the invention relates to a needle 14, which is capable of creating an electrical current upon insertion into a patient's body, and which exhibits increased conspicuity under MRI or CT due to the automatic generation of tiny bubbles of electrolytic gas. The needle 14 can be inserted into the patient's body under MRI or CT guidance using the apparatus 2 shown in Figure 1, and is clearly visible on an MRI or CT image as shown in Figure 10 compared to the image viewed using a prior art needle (see Figure 9). Indeed, any instrument which has an electrolysis-induced coating of gas bubbles will appear to have a dark surrounding on MRI and CT images.

In one embodiment, the needle 14 can be a simple 'Chiba'-style needle for gaining percutaneous access to an organ, or for the purpose of getting a tissue sample by fine needle aspiration (FNA).

In another embodiment, the needle 14 could be a spring-loaded biopsy needle 14 capable of extracting a core of tissue when activated inside a target zone, as shown in Figures 2 and 8. In another embodiment, the needle 14 can be a specialised needle 14 capable of delivering a substance (for example, a cell division inhibitor/promoter for use in chemotherapy, or a gene therapy product or an antibiotic etc), an electrical current (e.g. radiofrequency, AC or DC), or some other means that is used to ablate a target tissue, for example a tumour. Such a needle 14 may have multiple self-expanding tines capable of ablating a large tissue volume. In yet another embodiment, the needle 14 is either attached to a battery 40 or has an integrated battery 40 (e.g. in its barrel), the battery 40 creating an anode and cathode for the generation of gas bubbles 34, when in use.

The primary function of the needle 14 is not to kill cells or inhibit their growth, but rather to improve the ease, simplicity and accuracy of needle 14 insertion and positioning under MRI or CT guidance. In this way, non-target tissue can be avoided during insertion and withdrawal, and procedures can proceed more quickly and with a much greater degree of operator confidence. Thus, the needle 14 will lead to a significant reduction in operation training costs, and will improve the ease, speed, safety and patient tolerance of the procedure.

### Example 2

The inventor believes that the needle 14 can be efficiently used in combination with MRI guidance in the field of breast localization, using the apparatus 2 as shown in Figure 1. The patient 6 lies in an MRI scanner 2 and images are taken which show a breast tumour. A special mesh guide (not shown) is placed next to the skin which permits accurate depiction of the best route to take in order to insert a localizing wire (not shown) into the tumour. The physician directs the needle 14 into the breast tumour by inserting it through the skin while the patient 6 is still in the scanner 2, and, by taking regular images while advancing the needle 14 a short distance at a time, the tip of the needle 14 can be positioned exactly at the desired position. Subsequently, a special hooked wire (not shown) is advanced through the needle 14 into the tumour. The hook engages the tumour tissue preventing the wire from slipping out. Following this procedure, the patient 2, with the wire still in place, undergoes surgery. The surgeon uses the localizing wire to find the tumour at operation. Hence, the needle 14 of the invention becomes visible during this procedure, allowing accurate placement into the tumour.

## Claims

1. A method of determining the position, in a subject into whom an instrument (14) has been introduced, which comprises an anode (24) and a cathode (26) and in which, in use, current flows between the anode (24) and cathode (26), liquid inside the body of the subject acting as an electrolyte thereby producing a plurality of gas bubbles which are either directly or indirectly detectable by MRI or CT, the method comprising the steps of:
(a) carrying out either a magnetic resonance imaging (MRI) or a computed tomography (CT) procedure;
(b) generating, respectively, either an MRI or a CT image; and
(c) determining the position of the instrument in the subject based, respectively, on the MRI or the CT image, said gas bubbles rendering said instrument more conspicuous in said image.

2. A method according to Claim 1, wherein the medical instrument (14) is a stent, catheter, port, drug eluting element, electrical stimulator, pacemaker, a breast localization wire or needle, therapeutic or other irradiating element, or other implantable or insertable medical instrument, the position of which should be monitored during or after a medical procedure.

3. A method according to any preceding claim, wherein the instrument (14) is a penetrating instrument for percutaneous insertion into a patient.

4. A method according to any preceding claim, wherein the instrument (14) is a needle, for example a biopsy or localisation needle.

5. A method according to any preceding claim, wherein the instrument (14) comprises a power source (40), which is arranged to generate electrical current which flows between the anode and cathode.

6. A method according to claim 5, wherein the power source (40) is integral with the instrument (14), or is electrically connected thereto via electrically conductive wires.

7. A method according to either claim 5 or 6, wherein the power source (40) comprises a battery.

8. A method according to any one of claims 5-7, wherein the power source (40) is compatible with MRI or CT apparatus, or contained within a housing which is MRI or CT compatible.

9. A method according to any one of claims 5-8, wherein the power source (40) is substantially non-magnetic.

10. A method according to any preceding claim, further comprising attaching said instrument (14) to, or inserting said instrument (14) into, a medical device, preferably wherein said medical device is otherwise invisible or poorly visible by MRI or CT respectively, the plurality of gas bubbles produced by said instrument (14) rendering the medical device more conspicuous.

11. A method according to any preceding claim, wherein the instrument (14) comprises or consists of a metallic material.

12. A method according to any one of claims 1-6, wherein the instrument (14) comprises or consists of a non-metallic material

13. A method according to Claim 12, wherein the instrument (14) is made of glass, plastic, ceramic, or other non-metallic composite.

## Patentansprüche

1. Verfahren zum Bestimmen der Position, in einer Person, in die ein Instrument (14) eingeführt wurde, das eine Anode (24) und eine Kathode (26) umfasst und bei dem bei der Verwendung ein Strom zwischen der Anode (24) und der Kathode (26) fließt, wobei Flüssigkeit innerhalb des Körpers der Person als ein Elektrolyt wirkt, wodurch eine Vielzahl von Gasblasen erzeugt werden, die entweder direkt oder indirekt durch MRT oder CT erkennbar sind, wobei das Verfahren die folgenden Schritte umfasst:
(a) Durchführen entweder eines Magnetresonanztomographie (MRT)-oder eines Computertomographie (CT)-Verfahrens;
(b) jeweiliges Erzeugen eines MRT- oder eines CT-Bildes; und
(c) Bestimmen der Position des Instruments in der Person jeweils basierend auf dem MRT- oder dem CT-Bild, wobei die Gasblasen das Instrument in dem Bild stärker hervorheben.

2. Verfahren nach Anspruch 1, wobei das medizinische Instrument (14) ein Stent, ein Katheter, ein Port, ein Medikamente freisetzendes Element, ein Elektrostimulator, ein Herzschrittmacher, ein Brust-Lokalisierungsdraht oder eine Brust-Lokalisierungsnadel, ein therapeutisches oder anderes Bestrahlungselement oder ein anderes implantierbares oder einführbares medizinisches Instrument ist, dessen Position während oder nach einem medizinischen Verfahren überwacht werden sollte.

3. Verfahren nach einem der vorhergehenden Ansprüche, wobei das Instrument (14) ein eindringendes Instrument zum perkutanen Einsetzen in einen Patienten ist.

4. Verfahren nach einem der vorhergehenden Ansprüche, wobei das Instrument (14) eine Nadel ist, beispielsweise eine Biopsie- oder Lokalisierungsnadel.

5. Verfahren nach einem der vorhergehenden Ansprüche, wobei das Instrument (14) eine Stromquelle (40) umfasst, die dazu vorgesehen ist, einen elektrischen Strom zu erzeugen, der zwischen der Anode und der Kathode fließt.

6. Verfahren nach Anspruch 5, wobei die Stromquelle (40) einteilig mit dem Instrument (14) ausgebildet ist oder mit diesem elektrisch über elektrisch leitende Drähte verbunden ist.

7. Verfahren nach Anspruch 5 oder 6, wobei die Stromquelle (40) eine Batterie umfasst.

8. Verfahren nach einem der Ansprüche 5 bis 7, wobei die Stromquelle (40) mit MRT- oder CT-Vorrichtungen kompatibel ist oder in einem Gehäuse enthalten ist, das MRT- oder CT-kompatibel ist.

9. Verfahren nach einem der Ansprüche 5 bis 8, wobei die Stromquelle (40) im Wesentlichen nicht magnetisch ist.

10. Verfahren nach einem der vorhergehenden Ansprüche, ferner umfassend das Anbringen des Instruments (14) an oder das Einführen des Instruments (14) in eine medizinische Vorrichtung, wobei die medizinische Vorrichtung vorzugsweise anderweitig durch MRT bzw. CT nicht sichtbar oder schlecht sichtbar ist, wobei die Vielzahl von Gasblasen, die von dem Instrument (14) erzeugt werden, die medizinische Vorrichtung stärker hervorheben.

11. Verfahren nach einem der vorhergehenden Ansprüche, wobei das Instrument (14) ein metallisches Material umfasst oder daraus besteht.

12. Verfahren nach einem der Ansprüche 1 bis 6, wobei das Instrument (14) ein nichtmetallisches Material umfasst oder daraus besteht.

13. Verfahren nach Anspruch 12, wobei das Instrument (14) aus Glas, Kunststoff, Keramik oder einem anderen nichtmetallischen Verbundstoff hergestellt ist.

## Revendications

1. Procédé de détermination de la position, chez un sujet dans le corps duquel un instrument (14) a été introduit, lequel comprend une anode (24) et une cathode (26) et dans lequel, à l'usage, un courant circule entre l'anode (24) et la cathode (26), d'un liquide à l'intérieur du corps du sujet agissant comme un électrolyte, produisant ainsi une pluralité de bulles de gaz qui sont directement ou indirectement détectables par IRM ou TDM, le procédé comprenant les étapes consistant à :
(a) réaliser une procédure d'imagerie par résonance magnétique (IRM) ou de tomodensitométrie (TDM) ;
(b) générer une image IRM ou TDM, respectivement ; et
(c) déterminer la position de l'instrument dans le corps du sujet sur la base de l'image IRM ou TDM, respectivement, lesdites bulles de gaz rendant ledit instrument plus visible dans ladite image.

2. Procédé selon la revendication 1, dans lequel l'instrument médical (14) est un stent, un cathéter, un orifice, un élément à élution de médicament, un stimulateur électrique, un stimulateur cardiaque, un fil ou une aiguille de localisation mammaire, un élément irradiant thérapeutique ou autre, ou un autre instrument médical implantable ou insérable, dont la position devrait être surveillée pendant ou après une procédure médicale.

3. Procédé selon une quelconque revendication précédente, dans lequel l'instrument (14) est un instrument pénétrant pour insertion percutanée dans le corps d'un patient.

4. Procédé selon une quelconque revendication précédente, dans lequel l'instrument (14) est une aiguille, par exemple une aiguille de biopsie ou de localisation.

5. Procédé selon une quelconque revendication précédente, dans lequel l'instrument (14) comprend une source d'alimentation (40), qui est agencée pour générer un courant électrique qui circule entre l'anode et la cathode.

6. Procédé selon la revendication 5, dans lequel la source d'alimentation (40) fait partie intégrante de l'instrument (14), ou est reliée électriquement à celui-ci par le biais de fils électriquement conducteurs.

7. Procédé selon la revendication 5 ou 6, dans lequel la source d'alimentation (40) comprend une batterie.

8. Procédé selon l'une quelconque des revendications 5 à 7, dans lequel la source d'alimentation (40) est compatible avec un appareil d'IRM ou de TDM, ou contenue à l'intérieur d'un boîtier qui est compatible avec l'IRM ou la TDM.

9. Procédé selon l'une quelconque des revendications 5 à 8, dans lequel la source d'alimentation (40) est sensiblement amagnétique.

10. Procédé selon une quelconque revendication précédente, comprenant en outre la fixation dudit instrument (14) à, ou l'insertion dudit instrument (14) dans, un dispositif médical, de préférence dans lequel ledit dispositif médical est par ailleurs invisible ou peu visible par IRM ou TDM, respectivement, la pluralité de bulles de gaz produites par ledit instrument (14) rendant le dispositif médical plus visible.

11. Procédé selon une quelconque revendication précédente, dans lequel l'instrument (14) comprend ou consiste en un matériau métallique.

12. Procédé selon l'une quelconque des revendications 1 à 6, dans lequel l'instrument (14) comprend ou consiste en un matériau non métallique.

13. Procédé selon la revendication 12, dans lequel l'instrument (14) est constitué de verre, de plastique, de céramique, ou d'un autre composite non métallique.
